# EUROPÄISCHE PATENTSCHRIFT

(11) **EP 3 393 427 B1**
(45) Veröffentlichungstag und Bekanntmachung des Hinweises auf die Patenterteilung: **08.09.2021**
(21) Anmeldenummer: 16809036.3
(22) Anmeldetag: 09.12.2016
(51) Int. Cl.: A61K 8/25, A61K 8/365, A61Q 15/00

(54) **VERFAHREN ZUR SCHWEISSREDUKTION**
METHOD OF REDUCING PERSPIRATION
PROCÉDÉ DE RÉDUCTION DE LA TRANSPIRATION

(30) Priorität: 23.12.2015 DE 102015226630
(43) Veröffentlichungstag der Anmeldung: 31.10.2018
(73) Patentinhaber: Beiersdorf AG, 20253 Hamburg (DE)
(72) Erfinder: GROTHEER, Elke, 25474 Bönningstedt (DE); LEHMBECK, Frank, 22848 Norderstedt (DE); KLAUCK, Robert, 21465 Reinbek (DE); LUND, Nadia, 21077 Hamburg (DE)
(74) Vertreter: Beiersdorf AG
(86) Internationale Anmeldenummer: PCT/EP2016/080376
(87) Internationale Veröffentlichungsnummer: WO 2017/108445

(56) Entgegenhaltungen:
- EP-A1- 0 816 253
- WO-A1-2015/121667
- CH-A- 470 183
- DE-A1-102004 063 728
- DE-U1-202007 003 056
- FR-A1- 2 977 151
- US-A1- 2006 171 973

## Beschreibung

Die vorliegende Erfindung betrifft ein Verfahren zur Verminderung oder Verhinderung der apoekkrinen Schweißbildung durch Auftragung von zwei kosmetischen Teilzubereitungen, dadurch gekennzeichnet, dass der Zeitraum zwischen Mischung der Teilzubereitungen und Auftragung der Mischung auf die Haut nicht mehr als fünf Minuten beträgt und wobei eine Teilzubereitung ein Alkalisilikat aufweist und die zweite Teilzubereitung ein oder mehrere pH-Regulatoren aufweist.

Als Schweiß wird ein von der Haut des Menschen über so genannte Schweißdrüsen abgesondertes wässriges Sekret bezeichnet. Es gibt drei Arten von Schweißdrüsen in der Haut, nämlich apokrine, ekkrine und apoekkrine Schweißdrüsen (Int J Cosmet Sci. 2007 Jun; 29(3):169-79).

Die ekkrinen Schweißdrüsen sind beim Menschen praktisch über den ganzen Körper verteilt und können beträchtliche Mengen eines klaren, geruchlosen Sekretes produzieren, das zu über 99% aus Wasser besteht. Im Gegensatz dazu kommen die apokrinen Schweißdrüsen nur in den behaarten Körperarealen der Achsel- und Genitalregion sowie an den Brustwarzen vor. Sie produzieren geringe Mengen eines milchigen Sekretes, das Proteine und Lipide enthält und chemisch neutral ist.

Das Schwitzen, auch als Transpiration bezeichnet, ist ein effektiver Mechanismus, um überschüssige Wärme abzugeben und damit die Körpertemperatur zu regulieren. Hierzu dient vor allem das volumenreiche wässrige Sekret der ekkrinen Drüsen, die beim Erwachsenen bis zu 2-4 Liter pro Stunde bzw. 10-14 Liter am Tag produzieren können.

Dem Schweiß - insbesondere dem Sekret der apokrinen Schweißdrüsen - wird darüber hinaus eine Signalwirkung über den Geruchssinn zugesprochen. Beim Menschen spielt der apokrine Schweiß insbesondere im Zusammenhang mit dem emotionalen oder stressbedingten Schwitzen eine Rolle.

Kosmetische Antitranspirantien oder Desodorantien/Deodorantien dienen dazu, Körpergeruch zu beseitigen bzw. deren Entstehung zu vermindern. Körpergeruch entsteht, wenn der an sich geruchlose frische Schweiß durch Mikroorganismen wie z.B. Staphylokokken und Corynebakterien zersetzt wird.

Im allgemeinen Sprachgebrauch erfolgt nicht immer ein klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruchs (Geruchsverbesserungsmittel). Den üblichen kosmetischen Desodorantien liegen unterschiedliche Wirkprinzipien zugrunde.

Gängige Wirkmechanismen hierzu sind antibakterielle Effekte, wie sie z.B. auch das nichtkolloidale Silber zeigt, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Reaktionen zu wohlriechenden Stoffen umgesetzt werden.

Schweißgeruch besteht zu einem Großteil aus verzweigt-kettigen Fettsäuren, die durch bakterielle Enzyme aus geruchlosem Schweiß freigesetzt werden. Klassische Deo-Wirkstoffe wirken dem entgegen, indem sie das Wachstum von Bakterien reduzieren. Häufig wirken die dabei zum Einsatz kommenden Substanzen jedoch unselektiv auch gegen nützliche Hautkeime und können bei empfindlichen Personen zu Hautirritationen führen.

Als klassische Antitranspirantien werden vor allem Aluminiumsalze oder Aluminium-/Zirkonium-Salze verwendet. Diese hemmen den Schweißfluss durch Verstopfung der Ausführungsgänge der Schweißdrüsen, indem sie vor Ort zusammen mit hauteigenen Proteinen ausfallen und so zu sogenannten Plugs führen. Daher kann es zu einem Stau des Schweißes innerhalb der Drüse kommen.

Die Wirkung von Antitranspirantien auf Basis von Al-Salzen gegen thermisches Schwitzen unter normalen physiologischen Bedingungen ist sehr gut untersucht.

Ob diese Verstopfung durch Denaturierung des Keratins oder durch Verklumpung von Korneozyten im Schweißdrüsengang verursacht wird (Shelley WB and Hurley HJ, Acta. Derm. Venereol. (1975) 55: 241-60), oder durch die Entstehung eines ACH/AZG-Gels (Reller HH and Luedders WL, in: Advances in Modern Toxicology, Dermatoxicology and Pharmocology, F.N. Marzulli and H.I. Maibach, Eds. Hemisphere Publishing Company, Washington and London (1977) Vol. 4: 1-5), das durch Neutralisation im Schweißdrüsenausführgang gebildet wird, ist nach wie vor offen.

Die so erzielte und bekannte Verstopfung ist allerdings nur kurzfristig wirksam. Starkes Schwitzen oder die Reinigung der Achsel im Rahmen der normalen Körperreinigungsroutine heben die Verstopfung wieder auf und somit auch den Antitranspiranteffekt. Die daraus resultierende Notwendigkeit, Antitranspirant (AT)-Produkte mindestens einmal täglich aufzutragen führt aber ggf. zu Hautreizungen, speziell nach der Rasur oder in oder an vorgeschädigten Hautarealen.

Darüber hinaus können solche Aluminiumsalze wie Aluminiumhydroxychloride bei häufiger Anwendung und empfindlichen Personen Hautschäden hervorrufen. Darüber hinaus kann es durch den Einsatz der Aluminiumsalze zu Verfärbungen von Textilien kommen, die mit dem Antitranspirans in Kontakt kommen.

Durch die zusätzliche Verwendung antimikrobieller Stoffe in kosmetischen Antitranspirantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt.

Üblicherweise werden Antitranspirantien (AT) und Deodorantien (Deo) in mannigfaltigen Produktformen angeboten, wobei in Europa Roller, Pumpzerstäuber und Aerosole dominieren, in den USA, Mittel- und Südamerika eher die Deo-Stifte ("Sticks"). Es sind sowohl wasserfreie (Suspensionen) als auch wasserhaltige Produkte (hydro-alkoholische Formulierungen, Emulsionen) bekannt.

An ein zufriedenstellendes Deo-Mittel werden folgende Voraussetzungen geknüpft: 1) Schonung der natürlichen Biologie der Haut 2) Duftneutralität 3) Wirksamkeit nur in Bezug auf Desodorierung, d.h. nur Vermeidung und/oder Beseitigung von Körpergeruch 4) Vermeidung der Bildung von resistenten Bakterienstämmen 5) Unschädlichkeit bei Überdosierung oder sonstiger nicht bestimmungsgemäßer Anwendung 6) Gute kosmetische Anwendung 7) Leichte Handhabung (z.B. als Flüssigkeit) und universelle Verwendbarkeit in verschiedensten kosmetischen und externen Zubereitungen. 8) Ausgezeichnete Haut- und Schleimhautverträglichkeit 9) Einsatz umweltfreundlicher Stoffe
Bekannt und gebräuchlich sind neben den flüssigen Desodorantien und Antitranspirantien auch feste Zubereitungen, beispielsweise Puder, Pudersprays als, Intimreinigungsmittel.

Die Art der Zusammensetzung kosmetischer Zubereitungen findet ihre natürlichen Grenzen, die durch die Verträglichkeit der Einzelkomponenten miteinander sowie der Stabilität von Einzelkomponenten im Trägermedium bestimmt werden. Auch die Kombination unterschiedlich geladener Polymerer oder Tenside führt in kosmetischen Formulierungen zu Verklumpung und Ausfällungen.

Es hat nicht an Versuchen gefehlt, derartige Zubereitungen dem Verbraucher zugänglich zu machen. Meist werden dann Teilkomponenten der Formulierungen getrennt verpackt und aufbewahrt. In der Regel werden hierzu sogenannte Zwei-Kammer-Packmittel (engl. dual chamber) verwendet, bei denen die Teilzubereitungen in getrennten Vorratsgefäßen aufbewahrt und dem Packungsbehältnis gleichzeitig über eine gemeinsame Öffnung entnommen werden können.

Die FR 2852928 offenbart ein Zweikammerbehältnis bei dem die Produktentnahme mittels zweier Pumpen in der Weise erfolgt, dass die Zubereitungen erst im Ausgabekanal gemischt werden.

Aus US 20040149775 ist ein Zweikammerbehältnis bei dem die Kammern austauschbar sind bekannt. Auch hier erfolgt die Ausgabe der Zubereitungen mittels zweier Pumpen über einen gemeinsamen Ausgabekanal in dem die Vermischung der Komponenten erfolgt.

EP 958062 offenbart ein Zweikammerpackmittel mit konzentrisch angeordneten Pumpen.

In der FR 2900550 werden verschiede Zweikammerpackmittel offenbart, die eine beflockte Applikatoroberfläche aufweisen. Die Mischung der Komponenten erfolgt erst direkt bei der Auftragung.

EP 0461010 offenbart ein Zweikammeraerosolpackmittel, bei dem die Mischung der Komponenten erst vor dem Austreten aus der Düse erfolgt.

Aus der US 20060054634 sind Mehrkammerbehältnisse bekannt, die nach den Bag-in-Can Prinzip funktionieren und bei den die mindestens zwei Kammern aus miteinander verbundenen Beuteln aufgebaut sind.

Im der Deodorant- und/oder Antitranspirantprodukte ist die Verwendung von Zweikammerpackmitteln noch unbekannt, da die bisher eingesetzten aluminiumhaltigen Formulierungen, in denen ACH als Feststoff vorliegt, außerordentlich stabil sind.

Nachteilig an den bislang zur Schweißhemmung verwendeten Aluminium-Salzen ist, die derzeit noch nicht vollständig geklärte Langzeittoxizität. Aluminium steht seit langem im Verdacht, neurodegenarative Krankheiten wie Demenz, insbesondere Alzheimer, zu fördern oder auszulösen. Auch steht wird Aluminium mit der Entstehung von Brustkrebs in Verbindung gebracht. Bisher gibt es keine gesicherten Nachweise, dass über die Haut wirkende aluminiumhaltige AT-Mittel daran beteiligt sind. Bei intakter Haut sind die maximal zulässigen Aufnahmemengen nicht zu erreichen.

In Hinblick auf die Datenlage ist jedoch die Abkehr von aluminumhaltigen AT-Mitteln von Vorteil, so dass die Industrie händeringend nach aluminiumfreien Alternativen sucht.

Aufgrund dieser Problemstellung ist es wünschenswert Produkte zur Verfügung zu stellen, die eine Antitranspirantwirkung ohne Verwendung von Al-Salzen erzielen.

Eine Alternative zu Al-Salzen stellen kurzkettige Silikate in Form von Kieselsäuren dar, die eine Schweißbildung zuverlässig unterdrücken können.

Aus WO 2015/121667 sind saure, stabilisierte Kieselsäuren als antitranspirante Wirkstoffe offenbart. Die Wirkung beruht auf einer Polymerisation der sauren Kieselsäuren, die durch eine Erhöhung des pH-Wertes hervorgerufen wird. Diese Erhöhung erfolgt durch Kontakt mit Haut und Schweiß. Die erforderlichen Stabilisatoren verlangsamen die Polymerisation der Kieselsäure. Die sauren, stabilisierten Kieselsäuren werden in einem speziellen Verfahren hergestellt.

Ein Nachteil der so hergestellten sauren Kieselsäure ist deren Unverträglichkeit mit vielen der üblichen Kosmetischen Hilfsstoffe wie Emulgatoren, Tenside und Ölen. Dadurch ist es nahezu nicht möglich die üblichen Formulierungsformen wie Emulsionen stabil herzustellen.

Da pH-Werte unter 3,0 physiologisch unverträglich sind, müssen Zubereitungsformen gefunden werden, bei denen der pH-Wert mindestens bei 3,0 liegt und bei denen keine Gelbildung bzw. Ausfällung amorpher Kolloide (Kieselsol) erfolgt. Das Irritationspotential hängt sehr stark davon ab wie stark die aufgetragene Zubereitung gepuffert ist. Ist die Zubereitung nur schwach gepuffert, wird der pH-Wert auf der Haut sehr schnell auf ein physilogisch verträgliches Maß erhöht (bzw. abgesenkt bei basischen Zubereitungen. Bei stark gepufferten Zubereitungen muss der pH-Wert der Zubereitung möglichst nahe am verträglichen pH-Wert von 5 bis 8 sein.

FR 2977151 beschreibt den Einsatz von alkalischen Silikaten als antitranspirante Wirkstoffe.

Die bekannten Kiselsäuren bzw. Silikate besitzen den Nachteil, dass sie entweder als saure oder alkalische Komponenten auf die Haut aufgetragen werden müssen. Das kann zu Hautreizungen und Unverträglichkeiten führen. Daher ist es vorteilhaft, Produkte mit einem hautfreundlichen pH-Wert einzusetzen und damit dem Verbraucherwunsch nach einem stark wirksamen und gleichzeitig hautfreundlichen oder gar pflegenden Antitranspirant nachzukommen. Außerdem ist es vorteilhaft, ein spezielles, aufwändiges Verfahren für die Herstellung der aktiven Substanzen zu vermeiden.

Wünschenswert wäre es demnach ein Antitranspirantprodukt zur Verfügung zu stellen, das die vorgenannten Nachteile und Nebenwirkungen, insbesondere der ACH-haltigen Zubereitungen, nicht aufzeigt.

Wünschenswert wäre es weiterhin, ein Antitranspirantprodukt zur Verfügung zu stellen, das den Stand der Technik bereichert und eine Alternative zu den bekannten Zubereitungen, insbesondere ACH-haltigen Zubereitungen, darstellt.

Insbesondere ist es auch wünschenswert ein Antitranspirantprodukt zur Verfügung zu stellen, das keine Hautreizung nach der Auftragung verursacht, also in einen physilogisch akzeptablen pH-Wert aufweist

Eine weitere Aufgabe der vorliegenden Erfindung war es also, ein Produkt zu entwickeln, welche als Grundlage für kosmetische Desodorantien bzw. Antitranspirantien geeignet sind, und die Nachteile des Standes der Technik nicht aufweisen, insbesondere sich durch gute Hautverträglichkeit auszeichnen.

Aufgabe der Erfindung ist es insbesondere, silikathaltige Antitranspirantzubereitungen enthaltend mindestens eine Alkalisilikat in Kombination mit einem oder mehreren pH-Regulator(en) zur Verfügung zu stellen, die einen physiologisch verträglichen pH-Wert aufweisen.

Es war nach all diesem überraschend und nicht vorhersehbar, dass silikathaltige Zubereitungen mit einem pH von mindestens 4 bis 8, enthaltend ein oder mehrere Alkalisilikate und ein oder mehrere pH-Regulator(en) zur Verwendung als hautverträgliche Antitranspirantien, also zur Verminderung oder Verhinderung der apoekkrinen Schweißbildung, eignen, wenn die Mischung der Komponenten erst kurz vor der Applikation erfolgt und dadurch die Nachteile des Standes der Technik, die mangelnde Stabilität von physiologisch verträglichen Silikat-Zubereitungen, beseitigt wird.

Es war erstaunlich, dass mittels getrennter Bereitstellung von einer silikathaltiger Zubereitung und einem pH-Regulator in einem Zweikammerpackmittel, wobei die silikathaltige Zubereitung mit dem pH-Regulator erst kurz vor oder während der Ausgabe und/oder Applikation gemischt werden, nicht nur für kosmetische Zwecke hervorragend geeignet sind, sondern überdies wirkungsvoller und schonender sind als die Verwendung von stabilen Zusammensetzungen des Standes der Technik.

Die Erfindung ist daher ein Verfahren zur Verminderung oder Verhinderung der apoekkrinen Schweißbildung dadurch gekennzeichnet, das in einem ersten Schritt zwei kosmetische Teilzubereitungen gemischt werden und die Mischung in einem zweiten Schritt auf die Haut appliziert wird, wobei der der Zeitraum zwischen Mischung der Teilzubereitungen und Auftragung der Mischung auf die Haut nicht mehr als fünf Minuten beträgt und wobei eine Teilzubereitung ein Alkalisilikat aufweist und die zweite Teilzubereitung einen oder mehrere pH-Regulator(en) aufweist, wobei der pH-Regulator gewählt wird aus der Gruppe Puffersysteme oder ungepufferte Säuren, insbesondere Puffersysteme aufweisen mindestens eine Substanz aus der Gruppe 2-Aminobutanol, 2-(2-Aminoethoxy)ethanol, Aminoethyl Propanediol, Aminomethyl Propanediol, Aminomethyl Propanol, Aminopropanediol, Bis- Hydroxyethyl Tromethamine, Butyl Diethanolamine, Butylethanolamine, Dibutyl Ethanolamine, Diethanolamine, Diethyl Ethanolamine, Diisopropanolamine, Dimethylamino Methylpropanol, Dimethyl Isopropanolamine, Dimethyl MEA, Ethanolamine, Ethyl Ethanolamine, Isopropanolamine, Methyl Diethanolamine, Methylethanolamine, Triethanolamine, Triisopropanolamine, Tromethamine, Polyethylenimine, Tetrahydroxypropyl ethylendiamin, Ammoniak und mindestens einen Substanz aus der Gruppe Zitronensäure, Milchsäure, Weinsäure, Fettsäuren, Phosphorsäure, Phosphonsäuren, Polyacrylsäuren, Bernsteinsäure, Äpfelsäure, Oxalsäure, Aminosäuren oder insbesondere mindestens eine ungepufferte Säure oder deren Salze aus der Gruppe Salzsäure, Schwefelsäure oder Phosphorsäure, wobei deren Anionen physiologisch verträglich und gut in Lösung zu halten sind, wobei Phase 1 und/oder Phase 2 weniger als 0,1 Gew.-% an Aluminiumverbindungen enthalten.

Die Erfindung ist über ein kosmetisches Produkt aufweisend ein Zweikammerbehältnis geeignet zur Applikation von kosmetischen Zubereitungen, dadurch gekennzeichnet, dass eine Kammer eine Zubereitung, enthaltend mindestens ein Alkalisilikat, beinhaltet (Phase 1) und die zweite Kammer eine Zubereitung, enthaltend mindestens einen pH-Regulator, beinhaltet (Phase 2), wobei bei der Applikation die Inhalte der ersten und zweiten Kammer gleichzeitig aus den Kammern entnommen werden und sich die Phasen 1 und 2 bei der Ausgabe oder kurz vor der Ausgabe mischen.

Eine schnelle pH-Wertreduktion ist ausschlaggebend für die erfolgreiche Bildung der Silikate bzw. Kieselsäuren. Erfolgt die pH-Wertreduktion zu langsam, bilden sich sehr hochmolekulare Kieselsäuren, welche keine Antitranspirantaktivität aufweisen, bis hin zur Gelbildung.

In Zusammenhang mit dem erfindungsgemäßen Verfahren wird immer postuliert das in der Mischung aus silikathaltiger Phase 1 und Phase 2 die Siliziumverbindungen weiterhin als Silikate vorliegen. Je nach pH-Wert der Mischung können jedoch auch Kieselsäuren vorliegen. Kieselsäure ist eine sehr schwache Säure mit einem pKs1 = 9,51; pKs2 = 11,74. Das heißt, im erfindungsgemäßen pH-Bereich liegt das, Silizium' überwiegend als Kieselsäure und nur wenig als Silikat vor.

Die nach diesem Verfahren hergestellten Mischungen sind nur kurzzeitig stabil, weshalb eine Anwendung dieser AT-Formulierung direkt nach dem Vermischen erforderlich ist. Erfolgt die Mischung nicht schnell genug oder erfolgt die Applikation zu spät, dann kann es zu einer Polymerisierung der Kieselsäure (Gelbildung) kommen. Diese hat dann keine AT-Wirkung mehr.

Die Erfindung umfasst demnach auch die Verwendung von Silikaten, als Antitranspirantwirkstoff, bevorzugt in topisch applizierbaren, insbesondere kosmetischen und/oder dermatologischen, Zubereitungen, wobei der pH-Wert der Zubereitung erst vor der Applikation auf ein physiologisch verträgliches Maß eingestellt wird, insbesondere der pH-Wert der applizierten Zubereitung nicht kleiner als 4 ist.

Der pH-Wert der aufgetragenen Mischung sollte zwischen 4 und 8, bevorzugt zwischen 5,1 und 6,9, besonders bevorzugt zwischen 5,3 und 6,7 liegen.

Die erfindungsgemäßen Mischungen zeigen eine antitranspirante Wirkung auch ohne eine weitere Änderung des pH-Wertes.

Die Silikate werden gewählt aus:
Alkalisilikat, insbesondere Natriumsilikat, Kaliumsilikat, Wasserglas, besonders bevorzugt Natriumsilikat
Vorteilhaft handelt es sich bei den Silikaten um Silikathaltige wässrige Lösungen der Silikate, wobei die Siliziumkonzentration im Bereich von 1 bis 10 Mol/l, bevorzugt im Bereich von 4 bis 8 Mol/l liegt.

Als Wasserglas werden aus einer Schmelze erstarrte, glasartige, also amorphe, wasserlösliche Natrium-, Kalium- und Lithiumsilicate oder ihre wässrigen Lösungen bezeichnet. Je nachdem, ob überwiegend Natrium-, Kalium- oder Lithiumsilicate enthalten sind, spricht man von Natronwasserglas, Kaliwasserglas oder Lithiumwasserglas.

Zur Herstellung fester Wassergläser (Festgläser) werden Gemenge aus Quarzsand und Kaliumcarbonat (für Kaliwasserglas) bzw. Natriumcarbonat (für Natronwasserglas) unter CO2-Entwicklung bei 1100 °C bis 1200 °C [5] verschmolzen:
Die von der Zusammensetzung der Gemenge abhängige, allgemeine Formel M2O · n SiO2 von technisch wichtigen Wassergläsern liegt etwa im Bereich zwischen n gleich 1 bis 4. In der Regel werden für ein Wasserglas das Mol- oder Massenverhältnis von SiO2 zu Na2O bzw. SiO2 zu K2O angegeben. Natronwasserglas (siehe auch Natriumsilicate) mit dem Molverhältnis 3,4 bis 3,5 bildet den mengenmäßig wichtigsten Anteil.
Das abgekühlte Glas wird zu einem Pulver gemahlen. Daraus wird durch Lösen in Wasser bei hohen Temperaturen (z. B. 150 °C bei 5 bar Druck) flüssiges Wasserglas (Flüssigglas) als klare, kolloide alkalische Lösung oder auch als alkalisches Gel (gallertartige bis feste Masse) gewonnen.

Die so gewonnenen silikathaltigen Lösungen sind stark alkalisch und weisen einen pH-Wert von >= 10 auf.

Eine alkalische Lösung von käuflich erhältlichem Natriumsilikat (Wasserglas) oder Kaliumsilikat (Kaliwasserglas). Diese hat z.B. einen Si-Gehalt von 6,25 Mol/l, was ca. 10,6 % Na₂O und ca. 26,5 % SiO2 entspricht.

Die Verwendung von Silikatlösungen ist sehr viel einfacher als die aus WO 2015/121667 bekannten Kieselsäuren, da Silikatlösungen nahezu unbegrenzt stabil sind und die sauren Kieselsäuren erst aus Silikaten mittels eines Kompliziert zu steuernden Verfahrens hergestellt werden müssen.

Die Verringerung des pH-Wertes wird mit Hilfe von pH-Regulatoren, enthaltend in Phase 2, erzielt.

Erfindungsgemäße pH-Regulatoren können Puffersysteme sein, die entweder in wässriger Lösung oder wasserfrei zugegeben werden. Die Puffersysteme können aus Basen, wie z.B.: 2-Aminobutanol, 2-(2-Aminoethoxy)ethanol, Aminoethyl Propanediol, Aminomethyl Propanediol, Aminomethyl Propanol, Aminopropanediol, Bis- Hydroxyethyl Tromethamine, Butyl Diethanolamine, Butylethanolamine, Dibutyl Ethanolamine, Diethanolamine, Diethyl Ethanolamine, Diisopropanolamine, Dimethylamino Methylpropanol, Dimethyl Isopropanolamine, Dimethyl MEA, Ethanolamine, Ethyl Ethanolamine, Isopropanolamine, Methyl Diethanolamine, Methylethanolamine, Triethanolamine, Triisopropanolamine, Tromethamine, Polyethylenimine, Tetrahydroxypropyl ethylendiamin, Ammoniak und kosmetisch akzeptablen Säuren bestehen und haben einen pH-Wert von 2 bis 6.

Beispiele für Säuren, die zur Pufferherstellung besonders geeignet sind, sind Zitronensäure, Milchsäure, Weinsäure, Fettsäuren, Phosphorsäure, Phosphonsäuren, Polyacrylsäuren, Bernsteinsäure, Äpfelsäure, Oxalsäure, Aminosäuren.

Als pH-Regulatoren können auch ungepufferte Säuren eingesetzt werden, es eignen sich insbesondere Mineralsäuren wie Salzsäure, Schwefelsäure oder Phosphorsäure, deren Anionen physiologisch verträglich und gut in Lösung zu halten sind. Die pH-Wertreduktion kann jedoch auch beliebige andere Säuren erfolgen, solange diese a) den pH-Wert entsprechend herabsetzen können und b) deren Salze, insbesondere Natriumsalz, physiologisch verträglich sind bzw. keine Hautreizungen verursachen. Bevorzugt wird zur pH-Wert-Herabsetzung Salzsäure verwendet.

Durch die zügige Mischung von Phase 1 und Phase 2 erfolgt eine Erniedrigung des pH-Wertes der Gesamtmischung auf einen pH-Wert zwischen 4 und 8, bevorzugt zwischen 5,1 und 6,9, besonders bevorzugt zwischen 5,3 und 6,7.

Zusätzlich können die Phase 1 (silikathaltige Phase) und die Phase 2 (pH-regulatorhaltige Phase) noch weitere für kosmetische Zubereitungen übliche Bestandteile wie Parfüm, Rheologiemodifizierer, Solubilisatoren, Pflegestoffe, Farbstoffe, Hautpflegekomponenten enthalten.

Vorteilhaft enthalten eine oder beide Komponenten Stabilisatoren, die die Polymerisation auf der Haut verzögern.

Die Stabilisatoren werden gewählt aus
- der Gruppe A: Hexenol cis 3 (CAS 928-96-1), Terpineol (CAS 8000-41-7), Linalool (CAS 78-70-6), Tetrahydrolinalool (CAS 78-69-3), Triethyl Citrate (CAS 77-93-0), 2-Isobutyl-4-hydroxy-4-methyltetrahydropyran (CAS 63500-71-0), Hexyl salicylate (CAS 6259-76-3), Phenylethyl alcohol (CAS 60-12-8), 3-Methyl-5-phenyl-1-pentanol (CAS 55066-48-3), 2,6-Dimethyl-7-octen-2-ol (CAS 18479-58-8), Benzyl salicylate (CAS 118-58-1), Geraniol (CAS 106-24-1), Citronellol (CAS 106-22-9) und Ethyllinalool (CAS 10339-55-6);
- der Gruppe B: Alkohole und Diole und
- der Gruppe C: Substanzen mit mindestens drei Hydroxylgruppen.

Insbesondere vorteilhaft sind Stabilisatoren aus der Gruppe Linalool (CAS 78-70-6), Benzyl salicylate (CAS 118-58-1), Geraniol (CAS 106-24-1) und Citronellol (CAS 106-22-9).

Insbesondere vorteilhafte Stabilisatoren aus der Gruppe B sind: Ethanol, 2-Propanol, PEG 8, Triethylenglycol, Methylphenylbutanol, Decandiol, Polyglyceryl-2-caprate, Oxalsäure.

Insbesondere vorteilhafte Stabilisatoren aus der Gruppe C sind: Sucrose (Mannose, Mannit), Glycerin, Pentaerithritol, Threitol, Erythritol, Hyaluronsäure.

Die topische Applikation von Mischungen aus Phase 1 und Phase 2 enthaltend Silikate in Kombination mit ein oder mehreren pH-Regulatoren und Stabilisatoren gewählt aus den Gruppe A, B und/oder C ermöglicht die Verminderung oder Verhinderung des Stress-Schwitzens.

Im Rahmen der Erfindung, wird als Antitranspirantwirkung die Möglichkeit der Verminderung oder Verhinderung der Schweißbildung verstanden. D.h. Silikate wirken als Schweißhemmer und vermindern die Schweißbildung und damit mittelbar auch den Schweißgeruch.

Durch die pH-Werterniedrigung der silikathaltigen Zubereitung bei bzw. vor der Ausbringung und Applikation, werden Zubereitungen zur Verfügung gestellt, die eine hohe physiologische Verträglichkeit aufweisen.

Die Mischung aus silikathaltigen Zubereitung mit der pH-Wert erniedrigenden Säure erst vor der Ausbringung aus einem Zweikammerpackmittel macht die Verwendung von silikathaltigen Zubereitungen als verträgliches Antitranspirantmittel erst möglich.

Erfindungsgemäße Produkte mit Silikaten, ermöglichen eine schweißhemmende Wirkung in der Größenordnung wie bekannte und bewährte Antitranspirantwirkstoffe, wobei die benötigte Konzentration an Silikaten sehr viel geringer ist als bei Verwendung von ACH.

Die pH-Werterniedrigung erst vor der Applikation führt auch zur Behebung der aufgeführten Nachteile, wie Hautreizung durch zu hohen bzw. zu niedrigen pH reiner Silikatlösungen bzw. nicht stabilisierter Kieselsäurelösungen und der in der Diskussion stehenden Toxizität von Aluminiumverbindungen.

Bevorzugt umfassen die Phasen des erfindungsgemäßen Verfahrens bzw. der verfahrensumsetzenden Produkte daher neben einer silikathaltigen Zubereitung, keine weiteren antitranspirant wirksamen Stoffe oder Zubereitungen, insbesondere keine Aluminiumsalze, insbesondere kein ACH und/oder AACH (aktiviertes Aluminiumchlorohydrat).

Verfahrensumsetzende Produkte sind Produkte bei deren Verwendung das erfindungsgemäße Verfahren ausgeführt wird.

Wesentlicher Vorteil der verfahrensumsetzenden Produkte ist darüber hinaus, dass sich gegenüber den auf Aluminiumsalzen basierenden AT-Mitteln, keinerlei Verfärbungen auf der Haut oder Kleidung zeigt. Das sogenannte Weißeln unterbleibt ebenso wie die nach mehrfachem Tragen und Waschen in direkt auf der Achselhaut aufliegenden Textilien zu beobachtenden Rückstände.

Die Silikate können auf einfache Weise in die zum erfindungsgemäßen Verfahren benötigten Phase 1 eingearbeitet werden. Bevorzugt werden sie als silikathaltige Lösung den übrigen Bestandteilen der Phase 1 zugesetzt. Der Anteil der silkathaltigen Lösung kann dabei bis zu 98% der Gesamtmenge der Formulierung ausmachen. Im einfachsten Fall werden der Silikatlösung nur ein Verdicker und ein Parfüm zugesetzt, wobei unter Parfüm eine Abmischung umfassend ein oder mehrere olfaktorisch wahrnehmbarer Einzelsubstanzen zu verstehen ist.

Entsprechend weisen die verfahrensumsetzenden kosmetischen Produkte, mindestens zwei getrennte Kammern auf.

Beispiele dafür sind insbesondere Zweikammeraerosolbehältern, Zweikammerquetschflaschen, Zweikammerbehältnisse mit einer Doppelpumpenvorrichtung oder Roll-on-Vorrichtungen (Auftragung über Bewegungskörper, zum Beispiel Kugel oder Rolle), wobei eine Kammer eine silikathaltige Phase enthält und die zweite Kammer den pH-regulatorhaltige Phase enthält.

Eine gute Methode ist auch das Verstreichen bzw. Verreiben mittels flächiger Applikatoren, die von einem Zweikammerbehältnis gespeist werden, insbesondere Applikatoren mit beflockter und/oder textiler Oberfläche, da diese eine geringe Verstopfungsneigung aufweisen.

Vorteilhaft, gegenüber aluminiumchlorohydrathaltigen AT-Sprays ist, das die Silikate in der Phase 1 gelöst vorliegen und nicht vor der Verwendung des Sprays durch Schütteln resuspendiert werden müssen. Die Verstopfungswahrscheinlichkeit der Düsen wird dadurch verringert.

Weiterhin ist ein verfahrensgemäßer Einsatz in Zweikammer-Roll-ons und Zweikammerpumpsprays möglich und vorteilhaft.

Pump-Sprays bieten wie die Aerosolsprays einen berührungslosen Auftrag der AT-Zubereitung auf die Haut. Bei Pump-Sprays kann jedoch auf druckfeste Behälter verzichtet werden. Zweikammerpumpsprays lassen sich metallfrei, insbesondere aluminiumfrei gestalten. Vorteilhaft sind zum Beispiel Behältnisse aus PE, PP oder PET, die mit einer oder zwei metallfreien Zerstäuberpumpe(n) verschlossen sind, wobei metallfrei bedeutet, dass die gepumpte Zubereitung nicht mit metallischen Bauteilen in Berührung kommt. Je nachdem ob nur eine Pumpe verwendet wird oder zwei Pumpen verwendet werden erfolgt die Zuführung über einen oder zwei Steigrohre und die Mischung der NPK-haltigen Zubereitung (Phase 1) mit der Base (Phase 2) findet vor oder nach der Pumpe/den Pumpen statt.

Aerosolspray: Die Silikate (Phase 1) werden in den erfindungsgemäßen Antitranspirantformulierungen bevorzugt in einer Menge von 0,1 bis 15 Gew.-%, berechnet als SiO₂, bezogen auf die Gesamtmasse der Zubereitung, d.h. inklusive der ggf. vorhandenen Treibgase, eingesetzt. Insbesondere Vorteilhaft sind Konzentrationen von 0,5 bis 5 Gew.-%.

Der Gehalt an Silizium/-verbindungen wird im Sinne der Erfindung als Gehalt an SiO₂ angegeben und berechnet, da das Silizium nicht als einheitliche Verbindung in den Phasen und in der Mischung vorkommt. Der SiO₂-Gehalt ist z.B. durch Veraschung gravimetrisch sehr leicht ermittelbar.

Als Wirkstofflösung wird die Summe aller Bestandteile ohne das Treibgas bezeichnet, da das Treibgas in der Regel erst bei der Abfüllung hinzukommt.

Im Sinne der Erfindung ist es auch, das die Phasen enthaltend Silikate (Phase 1) oder die pH-Regulatoren (Phase 2) kosmetische Hilfsstoffe enthalten, wie sie üblicherweise in kosmetischen Zubereitungen verwendet werden, z.B. Konservierungsmittel, Konservierungshelfer, Bakterizide, Parfüme, UV-Filter, Antioxidantien, wasserlösliche Vitamine, Mineralstoffe, suspendierte Festkörperpartikel, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Elektrolyte, organische Lösungsmittel, Alkohole, Polyole, Emulgatoren, Polymere, Schaumstabilisatoren oder Silikonderivate.

Bevorzugt werden die Zubereitungen optisch ansprechend transparent hergestellt und verwendet.

Die Phase 2 ist vorteilhaft dadurch gekennzeichnet, dass sie in Form einer wässrigen oder wässrig-alkoholischen Lösung, oder einer wasserfreien Zubereitung vorliegt.

Vorteilhaft können Phase 1 und Phase 2 auch Desodorantien zugesetzt werden.

Den üblichen kosmetischen Desodorantien liegen unterschiedliche Wirkprinzipien zugrunde. Durch die Verwendung antimikrobieller Stoffe als kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt. Auch die Kombination von Adstringenzien mit antimikrobiell wirksamen Stoffen in ein und derselben Zusammensetzung ist gebräuchlich.

Alle als Desodorantien gängigen Wirkstoffe können vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol), Ethylhexylglycerin, Phenoxyethynol, Pirocton Olamin, Koffein sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Ebenso kann ein antimikrobieller Silbercitratkomplex, wie er in der DE 202008014407 beschrieben ist, bevorzugt als desodorierender Bestandteil in Verbindung mit NPK eingesetzt werden.

Bevorzugt enthalten Phase 1 und/oder Phase 2 auch Polymere enthalten. Die Polymere stammen bevorzugt aus dem Bereich der Cellulosen und/oder der Polystyrole. Sie sind vorteilhaft hydrophob oder hydrophil modifiziert. Sie dienen der Viskositätseinstellung der Phasen und erleichtern die Pump- und Mischbarkeit, sowie das Ablauf- bzw. Verteilungsverhalten auf der Haut.

Als übliche kosmetische Inhaltsstoffe der Phase 2 des erfindungsgemäßen Produktes können neben Wasser, Ethanol und Isopropanol, Glycerin und Propylenglykol hautpflegende Fett- oder fettähnliche Stoffe sowie Öle, wie Ölsäuredecylester, Cetylalkohol, Cetylstearylalkohol und 2-Octyldodecanol, in den für solche Präparate üblichen Mengenverhältnissen sein, sowie schleimbildende und filmbildende Stoffe und Verdickungsmittel, z.B. Hydroxyethyl- oder Hydroxypropylcellulose, Polyacrylsäure, Polyvinylpyrrolidon und Wachse.

Aus den für kosmetische Zubereitungen bekannten Emulgatoren haben sich diese als vorteilhaft für die erfindungsgemäß verwendbaren Zubereitungen der Phase 2 herausgestellt:
Polyoxyethylen(20)-sorbitan-monolaurat, Polyoxyethylen(20)sorbitan monopalmitate, Polyoxyethylen(20)-sorbitan-monostearat, Polyoxyethylen(20)-sorbitan-monooleat, Sorbitan Trioleate, Polyglyceryl-10 Stearate, Polyglyceryl-4 Caprate, Lauryl Glucoside, Polyglyceryl-2 Dipolyhydroxylstearate, Polyglyceryl-10 Laurate, Polyglyceryl-4 Laurate, Decyl Glucoside,Propylene Glycol Isostearate, Glycol Stearate), Glyceryl Isostearate), Sorbitan Sesquioleate, Glyceryl Stearate, Lecithin, Sorbitan Oleate, Sorbitan Monostearate NF, Sorbitan Stearate, Sorbitan Isostearate, Steareth-2, Oleth-2, Glyceryl Laurate, Ceteth-2, PEG-30 Dipolyhydroxystearate, Glyceryl Stearate SE, Sorbitan Stearate (and) Sucrose Cocoate, PEG-4 Dilaurate, PEG-8 Dioleate, Sorbitan Laurate, PEG-40 Sorbitan Peroleate, Laureth-4, PEG-7 Glyceryl Cocoate, PEG-20 Almond Glycerides, PEG-25 Hydrogenated Castor Oil, Stearamide MEA, Glyceryl Stearate + PEG-100 Stearate, Polysorbate 85, PEG-7 Olivate, Cetearyl Glucoside, PEG-8 Oleate, Polyglyceryl-3 Methyglucose Distearate, PG-10 Stearate, Oleth-10, Oleth-10 / Polyoxyl 10 Oleyl Ether NF, Ceteth-10, PEG-8 Laurate, Ceteareth-12, Cocamide MEA, Polysorbate 60 NF, Polysorbate 60, PEG-40 Hydrogenated Castor Oil, Polysorbate 80, Isosteareth-20, PEG-60 Almond Glycerides, Polysorbate 80 NF, PEG-150 Laurate, PEG-20 Methyl Glucose Sesquistearate, Ceteareth-20, Oleth-20, Steareth-20, Steareth-21,Ceteth-20, Isoceteth-20, PEG-30 Glyceryl Laurate, Polysorbate 20, Polysorbate 20 NF, Laureth-23, PEG-100 Stearate, Steareth-100, PEG-80 Sorbitan Laurate.

Bevorzugt werden Glyceryl Isostearate, Glyceryl Stearate, Steareth-2, Ceteareth-20, Steareth-21, PEG-40 Hydrogenated Castor Oil, PG-10 Stearate, Isoceteth-20, Isosteareth-20 und Ceteareth-12 eingesetzt.

Als Lösungsvermittler bekannt, aber als für die erfindungsgemäß verwendbaren Zubereitungen der Phase 2 als Emulgatoren einsetzbar, sind des Weiteren bevorzugt zu wählen PEG-40 Hydrogenated Castor Oil, Polysorbate 80, Laureth-23, PEG-150 Laurate und PEG-30 Glyceryl Laurate.

Neben bzw. anstelle nichtionischer Emulgatoren sind auch kationische Emulgatoren geeignet, um stabile Formulierungen mit der erfindungsgemäßen Poylquaternium Polymeren zu erstellen. Bevorzugte geeignete kationische Emulgatoren sind zu wählen aus der Gruppe Cetrimonium Chloride, Palmitamidopropyltrimonium Chloride, Quaternium-87, Behentrimonium Chloride, Distearoylethyl Dimonium Chloride, Distearyldimonium Chloride, Stearamidopropyl Dimethylamin und/oder Behentrimonium Methosulfat.

Es ist ebenfalls vorteilhaft, den Zubereitungen der Phase 2 im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern die kosmetische oder dermatologische Zubereitung der Phase 2 eine Lösung oder Emulsion oder Dispersion darstellt, können als Lösungsmittel, Konsistenzgeber und/oder hauptpflegende Wirkstoffe verwendet werden:
- Wasser oder wässrige Lösungen
- Öle, wie Triglyceride der Caprin- oder der Caprylsäure und Alkylbenzoat, vorzugsweise aber cyclische Silikonöle oder leicht flüchtige Kohlenwasserstoffe;
- -Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; Pflanzliche Öle wie z.B. Avocadoöl, Wiesenschaumkrautöl, Olivenöl, Sonnenblumenöl, Rapsöl, Mandelöl, Nachtkerzenöl, Kokosöl. Palmöl, Leinöl, Shea Butter.
- Alkohole, Diole oder Polyole niedriger C-Zahl, sowie deren Ether, insbesondere Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.
- Hautpflegende Substanzen wie z.B. Panthenol, Allantoin, Harnstoff, Harnstoffderivate, Guanidin, Ascorbinsäure, Glycerylglucose,

Insbesondere werden Gemische der vorstehend genannten Inhaltsstoffe verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Dimethylether, Distickstoffmonoxid, Kohlendioxid, Stickstoff und Druckluft sind vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bei Aerosolzubereitungen werden häufig Öle zugesetzt, die in der Wirkstofflösung mit dem Treibgas (Propan, Butan, Isobutan) mischbar sind, da ein Öl, das nicht mischbar ist, zu Niederschlägen führt, die im Glasaerosol dazu führen, dass die Wirkstoffpartikel nicht mehr aufzuschütteln sind.

Kosmetische Zubereitungen der Phase 2 können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel (Verdicker), z.B. Tamarindenmehl, Konjakmannan, Guaran, Hydroxypropylguar, Johannisbrotkernmehl, Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose, Carboxymethylcellulose oder ein Gemisch aus Polyethylenglykol und Polyethylenglykolstearat oder -distearat, enthalten. Das Verdickungsmittel ist in der Formulierung z.B. in einer Menge zwischen 0,1 und 40 Gew.-%, bevorzugt zwischen 0,5 und 25 Gew.-%, enthalten.

Ansonsten sind die üblichen Maßregeln für das Zusammenstellen von kosmetischen Formulierungen zu beachten, die dem Fachmann geläufig sind.

Es folgen vorteilhafte Ausführungsbeispiele der vorliegenden Erfindung.

Nachfolgende Beispiele erläutern die für das erfindungsgemäß Verfahren verwendbaren Phasen, deren Mischung und Auftragung zur Verminderung oder Verhinderung der Schweißbildung führt.

Die Zahlenangaben stellen Gewichtsanteile, bezogen auf die Gesamtmasse der Mischung, dar.

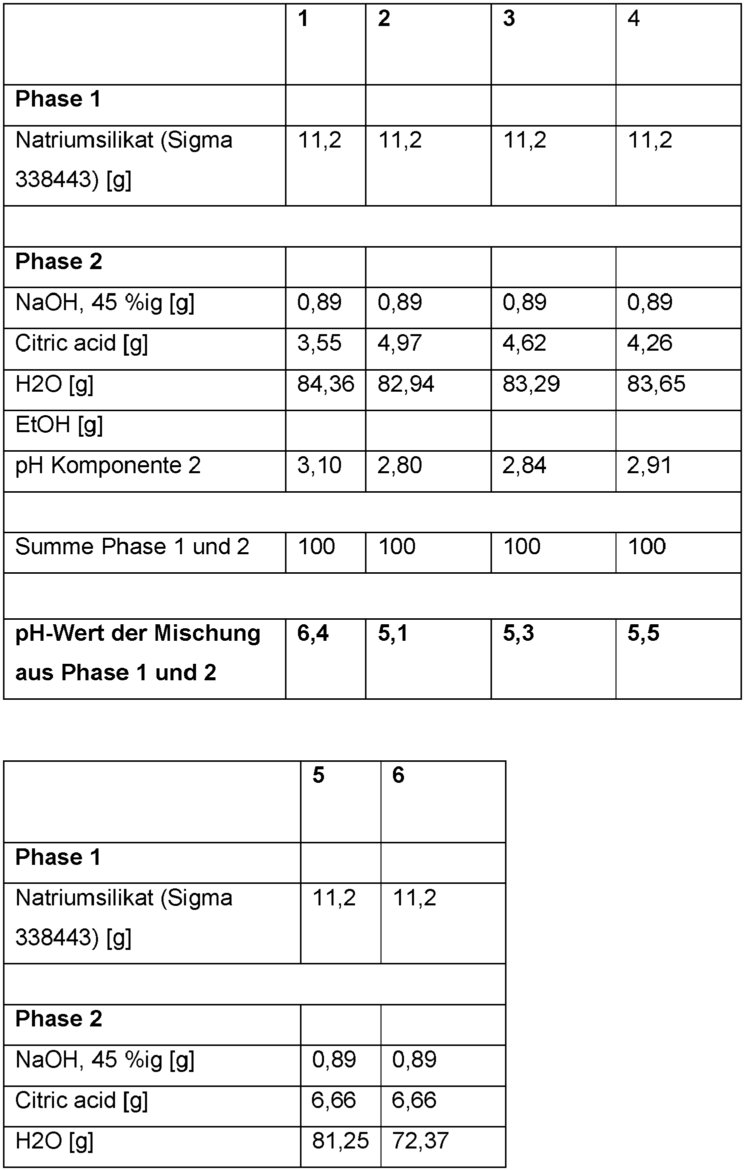

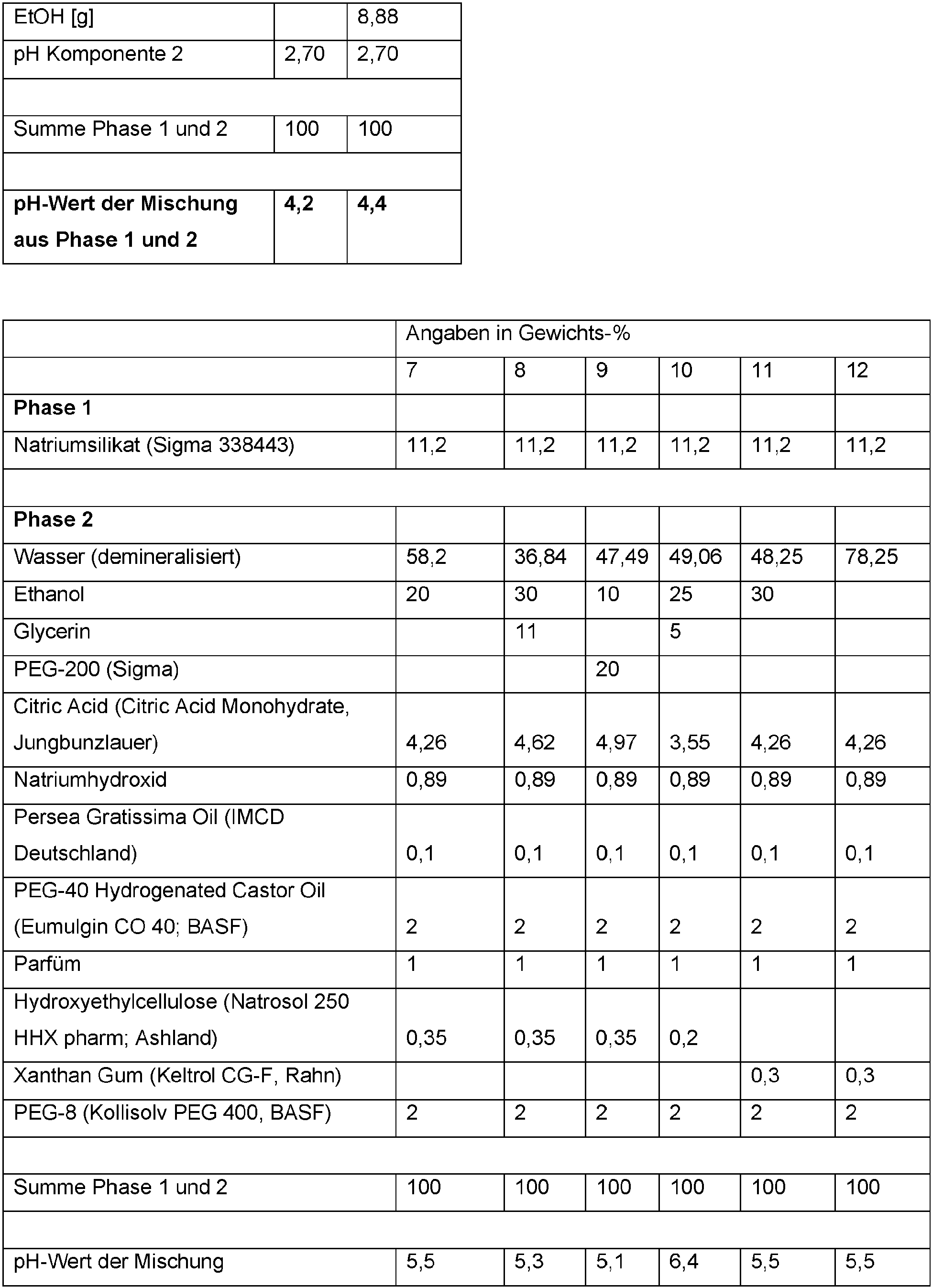

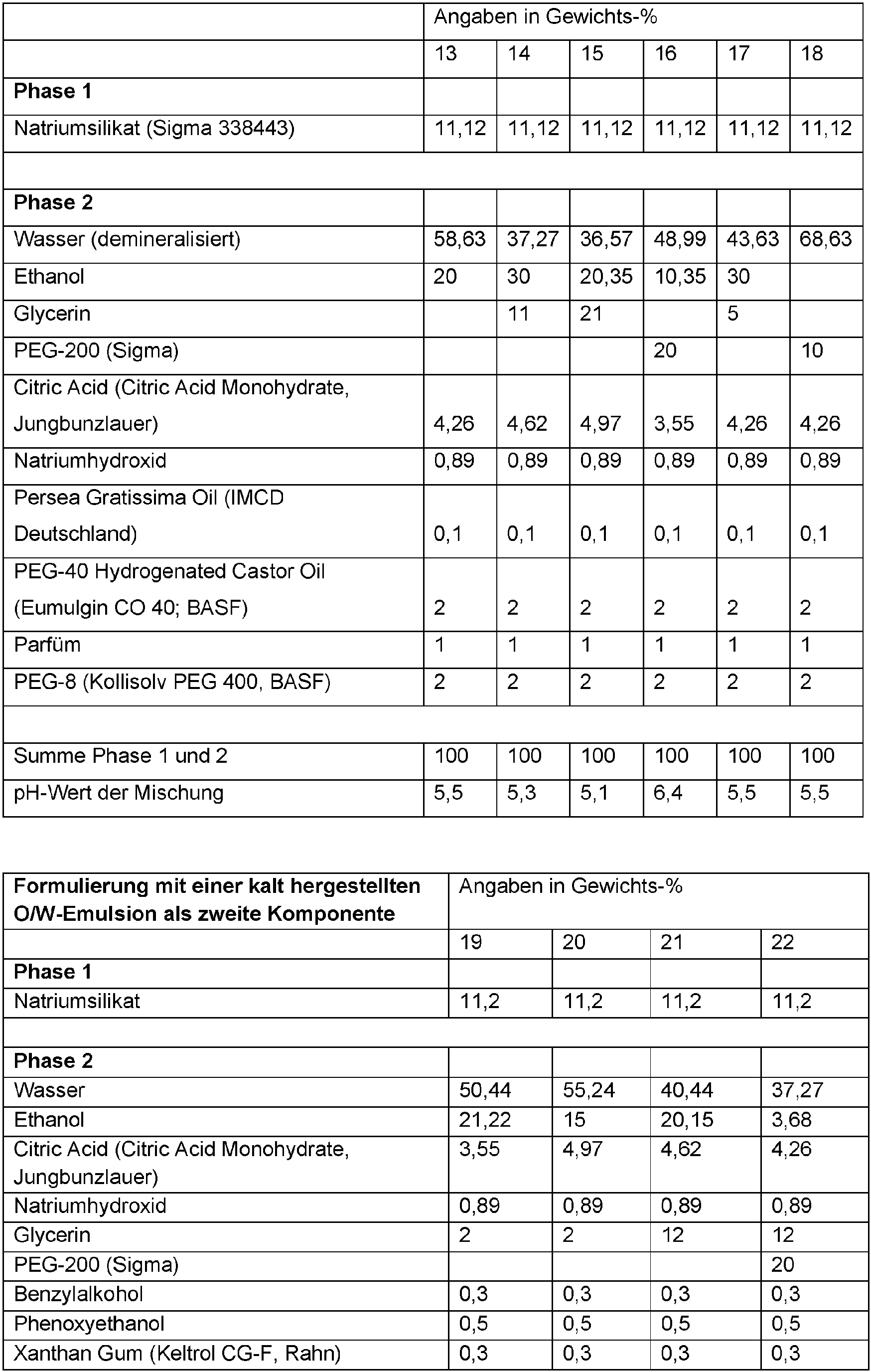

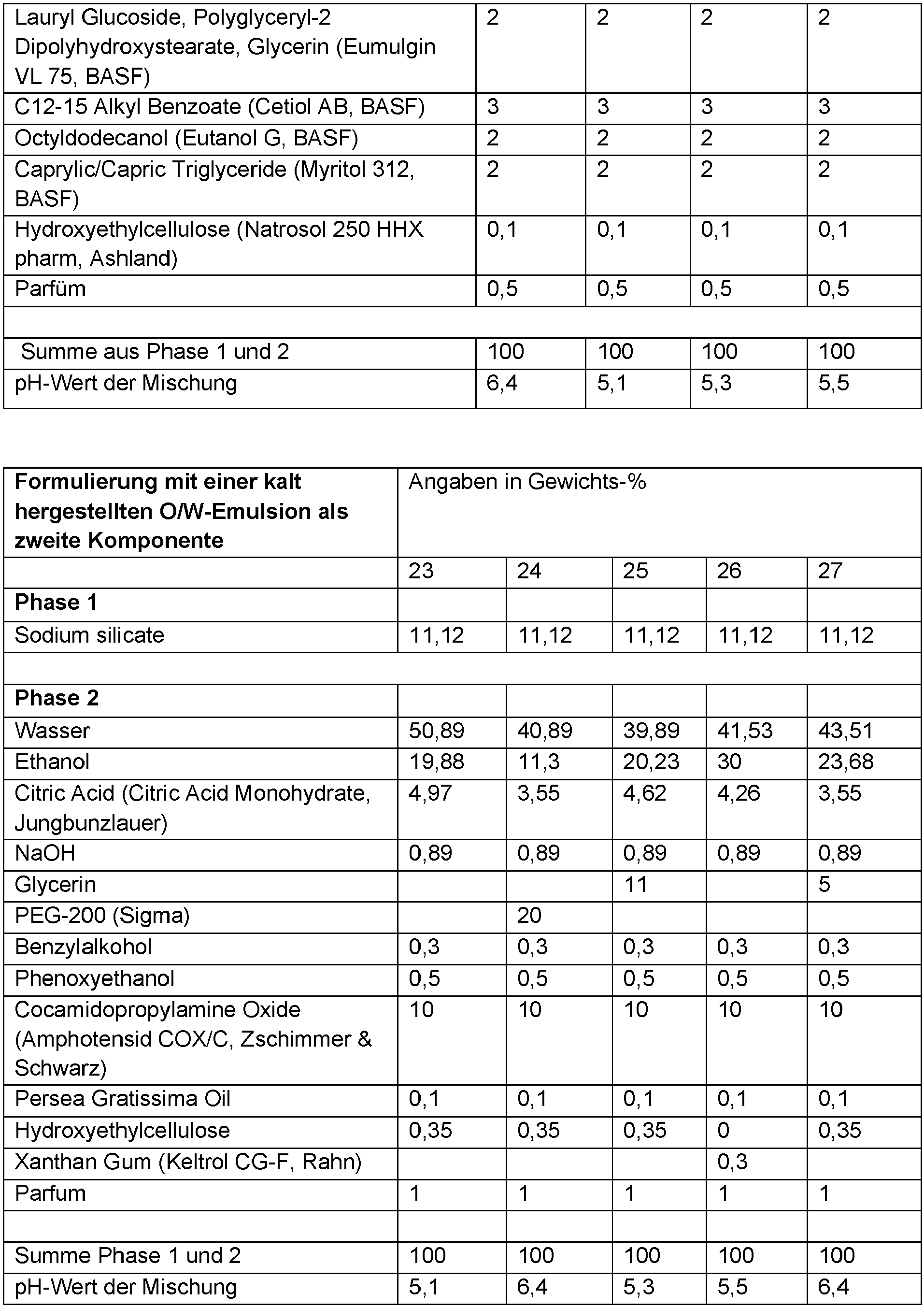

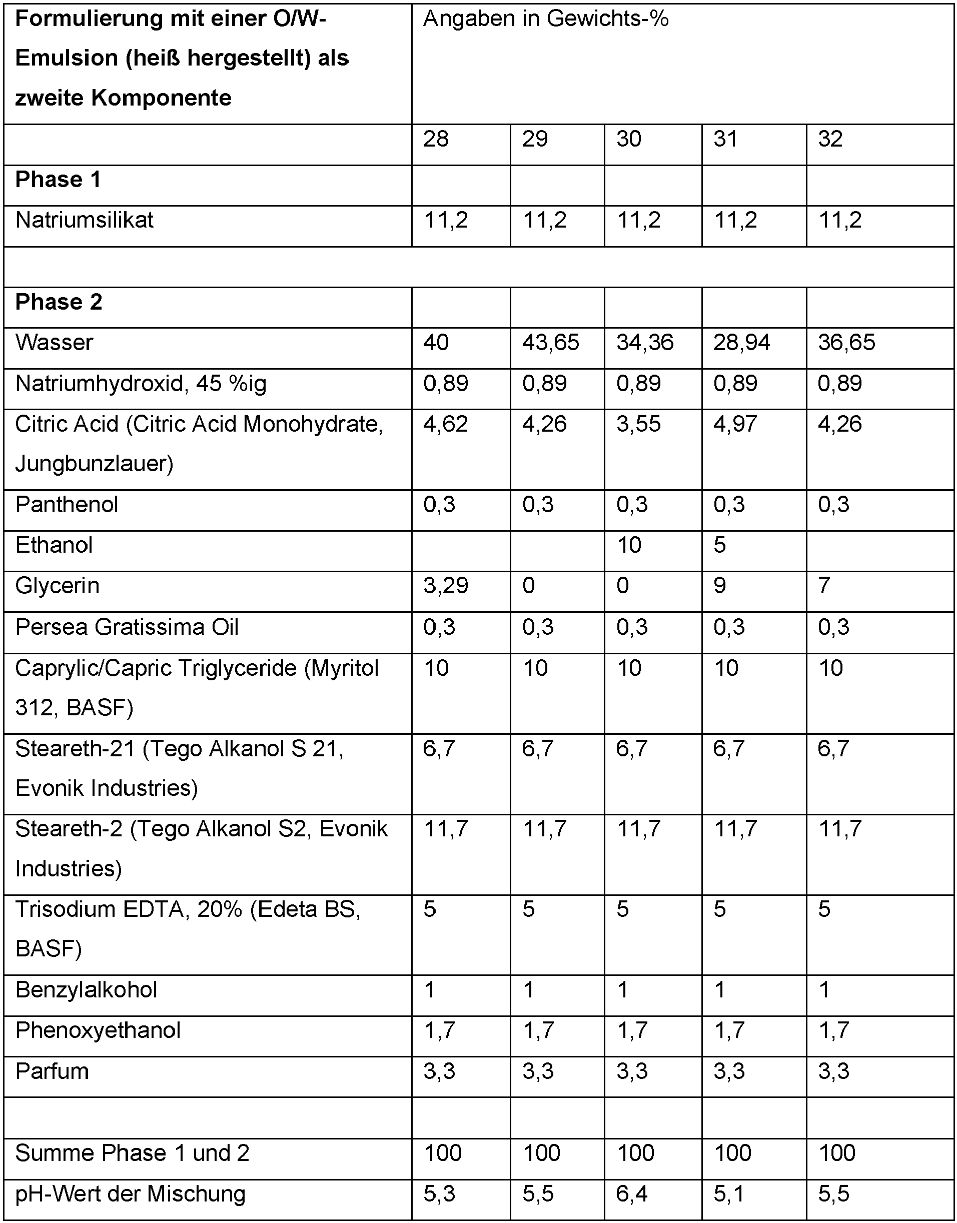

### Herstellung der Phase 2:

Die wasserlöslichen Bestandteile werden zusammen mit der angegebenen Menge Wasser auf 75 °C erwärmt. Die Ölkomponenten und Emulgatoren werden separat auf 75 °C erwärmt. Die Phasen werden vereinigt und homogenisiert. Die Emulsion wird unter Rühren auf Raumtemperatur abgekühlt.

## Patentansprüche

1. Verfahren zur Verminderung oder Verhinderung der apoekkrinen Schweißbildung durch Auftragung einer Mischung von zwei kosmetischen Teilzubereitungen, **dadurch gekennzeichnet, dass** der Zeitraum zwischen Mischung der Teilzubereitungen und Auftragung der Mischung auf die Haut nicht mehr als fünf Minuten beträgt und wobei eine Teilzubereitung ein oder mehrere Silikat(e) (Phase 1) aufweist und die zweite Teilzubereitung ein oder mehrere pH-Regulatoren (Phase 2) aufweist,
wobei der pH-Regulator gewählt wird aus der Gruppe Puffersysteme oder ungepufferte Säuren, insbesondere Puffersysteme aufweisen mindestens eine Substanz aus der Gruppe 2-Aminobutanol, 2-(2-Aminoethoxy)ethanol, Aminoethyl Propanediol, Aminomethyl Propanediol, Aminomethyl Propanol, Aminopropanediol, Bis- Hydroxyethyl Tromethamine, Butyl Diethanolamine, Butylethanolamine, Dibutyl Ethanolamine, Diethanolamine, Diethyl Ethanolamine, Diisopropanolamine, Dimethylamino Methylpropanol, Dimethyl Isopropanolamine, Dimethyl MEA, Ethanolamine, Ethyl Ethanolamine, Isopropanolamine, Methyl Diethanolamine, Methylethanolamine, Triethanolamine, Triisopropanolamine, Tromethamine, Polyethylenimine, Tetrahydroxypropyl ethylendiamin, Ammoniak und mindestens einen Substanz aus der Gruppe Zitronensäure, Milchsäure, Weinsäure, Fettsäuren, Phosphorsäure, Phosphonsäuren, Polyacrylsäuren, Bernsteinsäure, Äpfelsäure, Oxalsäure, Aminosäuren oder insbesondere mindestens eine ungepufferte Säure oder deren Salze aus der Gruppe Salzsäure, Schwefelsäure oder Phosphorsäure, wobei deren Anionen physiologisch verträglich und gut in Lösung zu halten sind,
wobei Phase 1 und/oder Phase 2 weniger als 0,1 Gew.-% an Aluminiumverbindungen enthalten.

2. Verfahren nach Anspruch 1, **dadurch gekennzeichnet**, das die Auftragung mittels eines Mehrkammerbehältnis erfolgt, wobei eine erste Kammer des Mehrkammerbehältnisses die Phase 1 beinhaltet und eine zweite Kammer des Mehrkammerbehältnisses die Phase 2 beinhaltet.

3. Verfahren nach Anspruch 1 oder 2, **dadurch gekennzeichnet, dass** das oder die Silikat(e) ein oder mehrere Alkalisilikat(e) ist/sind.

4. Verfahren nach Anspruch 3, **dadurch gekennzeichnet, dass** das Alkalisilikat Natriumsilikat ist.

5. Verfahren nach mindestens einem der vorhergehenden Ansprüche, **dadurch gekennzeichnet, dass** die Phase 1 einen Gehalt von 0,1 bis 15 Gew.-% an Siliziumhaltige Verbindungen, berechnet als SiO₂, bezogen auf die Gesamtmasse der Zubereitung (ggfs. inklusive der vorhandenen Treibgase) aufweist, insbesondere eine Konzentrationen von 0,5 bis 5 Gew.-% an Siliziumverbindungen.

6. Verfahren nach mindestens einem der vorhergehenden Ansprüche, **dadurch gekennzeichnet, dass** Phase 1 einen pH-Wert > 8, insbesondere > 9,5 aufweist.

7. Verfahren nach mindestens einem der vorhergehenden Ansprüche, **dadurch gekennzeichnet, dass** Phase 2 ein Parfüm enthält.

8. Verfahren nach mindestens einem der vorhergehenden Ansprüche, **dadurch gekennzeichnet, dass** Phase 1 und/oder 2 einen oder mehrere Stabilisator(en) gewählt aus der Gruppe Hexenol cis 3 (CAS 928-96-1), Terpineol (CAS 8000-41-7), Linalool (CAS 78-70-6), Tetrahydrolinalool (CAS 78-69-3), Triethyl Citrate (CAS 77-93-0), 2-Isobutyl-4-hydroxy-4-methyltetrahydropyran (CAS 63500-71-0), Hexyl salicylate (CAS 6259-76-3), Phenylethyl alcohol (CAS 60-12-8), 3-Methyl-5-phenyl-1-pentanol (CAS 55066-48-3), 2,6-Dimethyl-7-octen-2-ol (CAS 18479-58-8), Benzyl salicylate (CAS 118-58-1), Geraniol (CAS 106-24-1), Citronellol (CAS 106-22-9) und Ethyllinalool (CAS 10339-55-6), insbesondere aus der Gruppe Linalool (CAS 78-70-6), Benzyl salicylate (CAS 118-58-1), Geraniol (CAS 106-24-1) und Citronellol (CAS 106-22-9) enthält.

9. Verfahren nach mindestens einem der vorhergehenden Ansprüche, **dadurch gekennzeichnet, dass** Phase 1 und/oder 2 einen oder mehrere Stabilisator(en) gewählt aus der Gruppe der Alkohole und Diole, insbesondere aus der Gruppe: Ethanol, 2-Propanol, PEG 8, Triethylenglycol, Methylphenylbutanol, Decandiol, Polyglyceryl-2-caprate, Oxalsäure enthält.

10. Verfahren nach mindestens einem der vorhergehenden Ansprüche, **dadurch gekennzeichnet, dass** Phase 1 einen oder mehrere Stabilisator(en) gewählt aus der Gruppe der Substanzen mit mindestens drei Hydroxylgruppen, insbesondere aus der Gruppe:
Sucrose (Mannose, Mannit), Glycerin, Pentaerithritol, Threitol, Erythritol, Hyaluronsäure enthält.

11. Verfahren nach mindestens einem der vorhergehenden Ansprüche, **dadurch gekennzeichnet, dass** Phase 1 und/oder Phase 2 weniger als 0,05 mol/l an Aluminiumionen enthalten, bevorzugt beide Phasen frei von Aluminiumchlorohydrat sind.

12. Verfahren nach mindestens einem der vorhergehenden Ansprüche, **dadurch gekennzeichnet, dass** Phase 2 mindestens ein physiologisch verträgliches und/oder kosmetisches Öl und mindestens einen physiologisch verträglichen und/oder kosmetischen Emulgator enthält.

13. Kosmetisches Produkt zur Ausführung des Verfahrens nach mindestens einem der Ansprüche 1 bis 12, **dadurch gekennzeichnet, dass** das Produkt ein Mehrkammerbehältnis, insbesondere Zweikammerbehältnis, aufweist, wobei die erste Kammer eine Zubereitung umfassend ein oder mehrere Silikat(e) enthält (Phase 1) und die zweite Kammer eine Zubereitung umfassend ein oder mehrere pH-Regulatoren enthält (Phase 2) und bei der Applikation die in der ersten und zweiten Kammer gespeicherten Phasen 1 und 2 gleichzeitig aus den Kammern entnommen werden und sich die Phasen 1 und 2 bei der Ausgabe oder kurz vor der Ausgabe mischen, wobei der pH-Regulator gewählt wird aus der Gruppe Puffersysteme oder ungepufferte Säuren, insbesondere Puffersysteme aufweisen mindestens eine Substanz aus der Gruppe 2-Aminobutanol, 2-(2-Aminoethoxy)ethanol, Aminoethyl Propanediol, Aminomethyl Propanediol, Aminomethyl Propanol, Aminopropanediol, Bis- Hydroxyethyl Tromethamine, Butyl Diethanolamine, Butylethanolamine, Dibutyl Ethanolamine, Diethanolamine, Diethyl Ethanolamine, Diisopropanolamine, Dimethylamino Methylpropanol, Dimethyl Isopropanolamine, Dimethyl MEA, Ethanolamine, Ethyl Ethanolamine, Isopropanolamine, Methyl Diethanolamine, Methylethanolamine, Triethanolamine, Triisopropanolamine, Tromethamine, Polyethylenimine, Tetrahydroxypropyl ethylendiamin, Ammoniak und mindestens einen Substanz aus der Gruppe Zitronensäure, Milchsäure, Weinsäure, Fettsäuren, Phosphorsäure, Phosphonsäuren, Polyacrylsäuren, Bernsteinsäure, Äpfelsäure, Oxalsäure, Aminosäuren oder insbesondere mindestens eine ungepufferte Säure oder deren Salze aus der Gruppe Salzsäure, Schwefelsäure oder Phosphorsäure, wobei deren Anionen physiologisch verträglich und gut in Lösung zu halten sind und wobei Phase 1 und/oder Phase 2 weniger als 0,1 Gew.-% an Aluminiumverbindungen enthalten.

14. Verwendung eines Produktes nach mindestens einem der vorhergehenden Ansprüche, **dadurch gekennzeichnet dass** die kosmetische und/oder dermatologische Zubereitung als Aerosol oder mittels eines Bewegungskörpers topisch appliziert wird.

15. Verwendung eines Produktes nach mindestens einem der vorhergehenden Ansprüche, **dadurch gekennzeichnet dass** die kosmetische und/oder dermatologische Zubereitung mittels Verreiben bzw. Versteichen topisch appliziert wird.

## Claims

1. Method for reducing or preventing apoeccrine sweating by applying a mixture of two cosmetic part preparations, **characterized in that** the time period between mixing the part preparations and applying the mixture to the skin is not more than five minutes and wherein one part preparation comprises one or more silicate(s) (phase 1) and the second part preparation comprises one or more pH regulators (phase 2),
wherein the pH regulator is selected from the group comprising buffer systems or unbuffered acids, especially buffer systems comprising at least one substance from the group of 2-aminobutanol, 2-(2-aminoethoxy)ethanol, aminoethylpropanediol, aminomethylpropanediol, aminomethylpropanol, aminopropanediol, bishydroxyethyl tromethamine, butyldiethanolamine, butylethanolamine, dibutylethanolamine, diethanolamine, diethylethanolamine, diisopropanolamine, dimethylaminomethylpropanol, dimethylisopropanolamine, dimethyl MEA, ethanolamine, ethylethanolamine, isopropanolamine, methyldiethanolamine, methylethanolamine, triethanolamine, triisopropanolamine, tromethamine, polyethylenimine, tetrahydroxypropyl ethylenediamine, ammonia and at least one substance from the group comprising citric acid, lactic acid, tartaric acid, fatty acids, phosphoric acid, phosphonic acids, polyacrylic acids, succinic acid, malic acid, oxalic acid, amino acids or especially at least one unbuffered acid or salts thereof from the group comprising hydrochloric acid, sulfuric acid or phosphoric acid, wherein anions thereof are physiologically compatible and easy to keep in solution,
wherein phase 1 and/or phase 2 comprise less than 0.1% by weight of aluminium compounds.

2. Method according to Claim 1, **characterized in that** the application is effected by means of a multi-chamber container, wherein a first chamber of the multi-chamber container contains phase 1 and a second chamber of the multi-chamber container contains phase 2.

3. Method according to Claim 1 or 2, **characterized in that** the silicate (s) is/are one or more alkali metal silicate(s) .

4. Method according to Claim 3, **characterized in that** the alkali metal silicate is sodium silicate.

5. Method according to at least one of the preceding claims, **characterized in that** phase 1 comprises a content from 0.1 to 15% by weight of silicon-containing compounds, calculated as SiO₂, based on the total mass of the preparation (optionally including the propellant gases present), especially a concentration from 0.5 to 5% by weight of silicon compounds.

6. Method according to at least one of the preceding claims, **characterized in that** phase 1 has a pH > 8, especially > 9.5.

7. Method according to at least one of the preceding claims, **characterized in that** phase 2 comprises a perfume.

8. Method according to at least one of the preceding claims, **characterized in that** phase 1 and/or 2 comprises one or more stabilizer(s) selected from the group comprising cis-3-hexenol (CAS 928-96-1), terpineol (CAS 8000-41-7), linalool (CAS 78-70-6), tetrahydrolinalool (CAS 78-69-3), triethyl citrate (CAS 77-93-0), 2-isobutyl-4-hydroxy-4-methyltetrahydropyran (CAS 63500-71-0), hexyl salicylate (CAS 6259-76-3), phenylethyl alcohol (CAS 60-12-8), 3-methyl-5-phenyl-1-pentanol (CAS 55066-48-3), 2,6-dimethyl-7-octen-2-ol (CAS 18479-58-8), benzyl salicylate (CAS 118-58-1), geraniol (CAS 106-24-1), citronellol (CAS 106-22-9) and ethyl linalool (CAS 10339-55-6), more particularly from the group comprising linalool (CAS 78-70-6), benzyl salicylate (CAS 118-58-1), geraniol (CAS 106-24-1) and citronellol (CAS 106-22-9) .

9. Method according to at least one of the preceding claims, **characterized in that** phase 1 and/or 2 comprises one or more stabilizer (s) selected from the group of alcohols and diols, more particularly from the group: ethanol, 2-propanol, PEG 8, triethylene glycol, methylphenylbutanol, decanediol, polyglyceryl-2 caprate, oxalic acid.

10. Method according to at least one of the preceding claims, **characterized in that** phase 1 comprises one or more stabilizer(s) selected from the group of substances having at least three hydroxyl groups, more particularly from the group: sucrose (mannose, mannitol), glycerol, pentaerythritol, threitol, erythritol, hyaluronic acid.

11. Method according to at least one of the preceding claims, **characterized in that** phase 1 and/or phase 2 comprise less than 0.05 mol/l of aluminium ions, preferably both phases being free of aluminium chlorohydrate.

12. Method according to at least one of the preceding claims, **characterized in that** phase 2 comprises at least one physiologically compatible and/or cosmetic oil and at least one physiologically compatible and/or cosmetic emulsifier.

13. Cosmetic product for implementing the method according to at least one of claims 1 to 12, **characterized in that** the product comprises a multi-chamber container, more particularly a dual chamber container, wherein the first chamber comprises a preparation comprising one or more silicate(s) (phase 1) and the second chamber comprises a preparation comprising one or more pH regulators (phase 2) and during the application phases 1 and 2 stored in the first and second chamber are simultaneously withdrawn from the chambers and phases 1 and 2 are mixed on dispensing or shortly before dispensing, wherein the pH regulator is selected from the group comprising buffer systems or unbuffered acids, especially buffer systems comprising at least one substance from the group of 2-aminobutanol, 2-(2-aminoethoxy)ethanol, aminoethylpropanediol, aminomethylpropanediol, aminomethylpropanol, aminopropanediol, bishydroxyethyl tromethamine, butyldiethanolamine, butylethanolamine, dibutylethanolamine, diethanolamine, diethylethanolamine, diisopropanolamine, dimethylaminomethylpropanol, dimethylisopropanolamine, dimethyl MEA, ethanolamine, ethylethanolamine, isopropanolamine, methyldiethanolamine, methylethanolamine, triethanolamine, triisopropanolamine, tromethamine, polyethylenimine, tetrahydroxypropyl ethylenediamine, ammonia and at least one substance from the group comprising citric acid, lactic acid, tartaric acid, fatty acids, phosphoric acid, phosphonic acids, polyacrylic acids, succinic acid, malic acid, oxalic acid, amino acids or especially at least one unbuffered acid or salts thereof from the group comprising hydrochloric acid, sulfuric acid or phosphoric acid, wherein anions thereof are physiologically compatible and easy to keep in solution and wherein phase 1 and/or phase 2 comprise less than 0.1% by weight of aluminium compounds.

14. Use of a product according to at least one of the preceding claims, **characterized in that** the cosmetic and/or dermatological preparation is applied topically as an aerosol or by means of a moving body.

15. Use of a product according to at least one of the preceding claims, **characterized in that** the cosmetic and/or dermatological preparation is applied topically by means of rubbing or spreading.

## Revendications

1. Procédé de diminution ou de prévention de la transpiration apocrine par application d'un mélange de deux préparations partielles cosmétiques, **caractérisé en ce que** le laps de temps entre le mélange des préparations partielles et l'application du mélange sur la peau n'est pas supérieur à cinq minutes, et une préparation partielle comportant un ou plusieurs silicate(s) (Phase 1) et la seconde préparation partielle comportant un ou plusieurs régulateurs du pH (Phase 2),
le régulateur de pH étant choisi dans le groupe des systèmes tampons ou des acides non tamponnés, en particulier les systèmes tampons comprenant au moins une substance du groupe 2-aminobutanol, 2-(2-aminoéthoxy)éthanol, aminoéthylpropanediol, aminométhylpropanediol, aminométhylpropanol, aminopropanediol, bis-hydroxyéthyltrométhamine, butyldiéthanolamine, butyléthanolamine, dibutyléthanolamine, diéthanolamine, diéthyléthanolamine, diisopropanolamine, diméthylaminométhylpropanol, diméthylisopropanolamine, diméthyl MEA, éthanolamine, éthyléthanolamine, isopropanolamine, méthyldiéthanolamine, méthyléthanolamine, triéthanolamine, triisopropanolamine, trométhamine, polyéthylène-imine, tétrahydroxypropyléthylènediamine, ammoniac et au moins une substance du groupe acide citrique, acide lactique, acide tartrique, acides gras, acide phosphorique, acides phosphoniques, acides polyacryliques, acide succinique, acide malique, acide oxalique, acides aminés ou en particulier au moins un acide non tamponné ou ses sels du groupe acide chlorhydrique, acide sulfurique ou acide phosphorique, leurs anions étant physiologiquement compatibles et pouvant être bien maintenus en solution,
la Phase 1 et/ou la Phase 2 contenant moins de 0,1 % en poids de composés de l'aluminium.

2. Procédé selon la revendication 1, **caractérisé en ce que** l'application s'effectue à l'aide d'un récipient à plusieurs compartiments, un premier compartiment du récipient à plusieurs compartiments contenant la Phase 1 et le second compartiment du récipient à plusieurs compartiments contenant la Phase 2.

3. Procédé selon la revendication 1 ou 2, **caractérisé en ce que** le ou les silicate (s) sont un ou plusieurs silicate(s) de métaux alcalins.

4. Procédé selon la revendication 3, **caractérisé en ce que** le silicate d'un métal alcalin est le silicate de sodium.

5. Procédé selon au moins l'une des revendications précédentes, **caractérisé en ce que** la Phase 1 présente une teneur de 0,1 à 15 % en poids en composés contenant du silicium, calculés en SiO₂, par rapport à la masse totale de la préparation (éventuellement y compris les gaz propulseurs présents), en particulier une concentration de 0,5 à 5 % en poids de composés du silicium.

6. Procédé selon au moins l'une des revendications précédentes, **caractérisé en ce que** la Phase 1 présente un pH > 8, en particulier > 9,5.

7. Procédé selon au moins l'une des revendications précédentes, **caractérisé en ce que** la Phase 2 contient un parfum.

8. Procédé selon au moins l'une des revendications précédentes, **caractérisé en ce que** la Phase 1 et/ou la Phase 2 contiennent un ou plusieurs stabilisants choisis dans le groupe hexénol cis 3(CAS 928-96-1), terpinéol (CAS 8000-41-7), linalool (CAS 78-70-6), tétrahydrolinalool (CAS 78-69-3), citrate de triéthyle (CAS 77-93-0), 2-isobutyl-4-hydroxy-4-méthyltétrahydropyrane (CAS 63500-71-0), salicylate d'hexyle (CAS 6259-76-3), alcool phényléthylique (CAS 60-12-8), 3-méthyl-5-phényl-1-pentanol (CAS 55066-48-3), 2,6-diméthyl-7-octèn-2-ol (CAS 18479-58-8), salicylate de benzyle (CAS 118-58-1), géraniol (CAS 106-24-1), citronellol (CAS 106-22-9) et éthyllinalool (CAS 10339-55-6), en particulier du groupe linalool (CAS 78-70-6), salicylate de benzyle (CAS 118-58-1), géraniol (CAS 106-24-1) et citronellol (CAS 106-22-9).

9. Procédé selon au moins l'une des revendications précédentes, **caractérisé en ce que** la Phase 1 et/ou la Phase 2 contiennent un ou plusieurs stabilisants choisis dans le groupe des alcools et des diols, en particulier du groupe éthanol, 2-propanol, PEG 8, triéthylèneglycol, méthylphénylbutanol, décanediol, caprate de polyglycéryl-2, acide oxalique.

10. Procédé selon au moins l'une des revendications précédentes, **caractérisé en ce que** la Phase 1 contient un ou plusieurs stabilisant(s) choisis dans le groupe des substances ayant au moins trois groupes hydroxyle, en particulier du groupe saccharose (mannose, mannitol), glycérol, pentaérithritol, thréitol, érythritol, acide hyaluronique.

11. Procédé selon au moins l'une des revendications précédentes, **caractérisé en ce que** la Phase 1 et/ou la Phase 2 contiennent moins de 0,05 mol/l d'ions aluminium, de préférence les deux phases étant exemptes de chlorhydrate d'aluminium.

12. Procédé selon au moins l'une des revendications précédentes, **caractérisé en ce que** la Phase 2 contient au moins une huile physiologiquement tolérée et/ou cosmétique et au moins un émulsifiant physiologiquement toléré et/ou cosmétique.

13. Produit cosmétique pour la mise en œuvre du procédé selon au moins l'une des revendications 1 à 12, **caractérisé en ce que** le produit présente un récipient à plusieurs compartiments, en particulier un récipient à deux compartiments, le premier compartiment contenant une préparation comprenant un ou plusieurs silicates (Phase 1) et le second compartiment contenant une préparation comprenant un ou plusieurs régulateurs de pH (Phase 2), et, lors de l'application, les Phases 1 et 2 stockées dans le premier et dans le second compartiment étant prélevées des compartiments, les Phases 1 et 2 se mélangeant lors de la libération ou peu de temps avant la libération, le régulateur de pH étant choisi dans le groupe des systèmes tampons ou des acides non tamponnés, en particulier les systèmes tampons comprenant au moins une substance du groupe 2-aminobutanol, 2-(2-aminoéthoxy)éthanol, aminoéthylpropanediol, aminométhylpropanediol, aminométhylpropanol, aminopropanediol, bis-hydroxyéthyltrométhamine, butyldiéthanolamine, butyléthanolamine, dibutyléthanolamine, diéthanolamine, diéthyléthanolamine, diisopropanolamine, diméthylaminométhylpropanol, diméthylisopropanolamine, diméthyl MEA, éthanolamine, éthyléthanolamine, isopropanolamine, méthyldiéthanolamine, méthyléthanolamine, triéthanolamine, triisopropanolamine, trométhamine, polyéthylène-imine, tétrahydroxypropyléthylènediamine, ammoniac et au moins une substance du groupe acide citrique, acide lactique, acide tartrique, acides gras, acide phosphorique, acides phosphoniques, acides polyacryliques, acide succinique, acide malique, acide oxalique, acides aminés ou en particulier au moins un acide non tamponné ou ses sels du groupe acide chlorhydrique, acide sulfurique ou acide phosphorique, leurs anions étant physiologiquement compatibles et pouvant être bien maintenus en solution,
la Phase 1 et/ou la Phase 2 contenant moins de 0,1 % en poids de composés de l'aluminium.

14. Utilisation d'un produit selon au moins l'une des revendications précédentes, **caractérisée en ce que** la préparation cosmétique et/ou dermatologique est appliquée par voie topique sous forme d'un aérosol ou à l'aide d'un applicateur mobile.

15. Utilisation d'un produit selon au moins l'une des revendications précédentes, **caractérisée en ce que** la préparation cosmétique et/ou dermatologique est appliquée par voie topique, par application en frottant ou par étalement.
